# EUROPEAN PATENT APPLICATION

(11) **EP 4 722 704 A1**
(43) Date of publication of application: **08.04.2026**
(21) Application number: 25199282.2
(22) Date of filing: 01.09.2025
(51) Int. Cl.: G01N 27/12, G01N 27/22

(54) **MOISTURE IN FLUID SENSOR**

(30) Priority: 04.10.2024 IN 202411075199
(71) Applicant: HONEYWELL INTERNATIONAL INC., Charlotte, NC 28202 (US)
(72) Inventor: KISHORE, Kuna Venkat Satya Rama, Charlotte, 28202 (US); HALL, Jeff S., Charlotte, 28202 (US)
(74) Representative: Haseltine Lake Kempner LLP

(57) **Abstract**

A moisture detecting apparatus comprising an input port; an output port; a housing that is configured between the input port at a first end and the output port at a second end; an adsorbent material that is configured in a flow path of an inner cavity within the housing; and an electrical port comprising a controller that is coupled to the adsorbent material via one or more pin bars, wherein the controller is configured to (i) measure, using the one or more pin bars, one or more voltages on the adsorbent material and (ii) determine an electrical property of the adsorbent material based on the one or more voltages.

## Description

### TECHNICAL FIELD

Various embodiments of the present disclosure relate to moisture sensors, and more particularly to sensors that detect moisture in fluids.

### BACKGROUND

In process industries, such as oil and gas or heating, ventilation, and air conditioning (HVAC), a presence of moisture in process fluids (e.g., oils, refrigerants, fuels, gases, etc.) may pose challenges for overall control, efficiency, and reliability. For example, moisture in gas streams may introduce error in energy content which affects economies. In another example, moisture in hydraulic oils may cause long-term degradation of mechanical components. Similarly, moisture in transformer oils may reduce insulation strength and breakdown voltages. In yet another example, moisture in refrigeration systems may cause long term failure of compressors, expansion valves, and/or reduce overall energy efficiency. As such, controlling moisture levels in certain applications and/or environments may be critical.

Applicant has identified many technical challenges and difficulties associated with using conventional sensors to detect moisture in fluids.

### BRIEF SUMMARY

Various embodiments described herein relate to components, apparatuses, and systems for measuring moisture in a fluid system.

In accordance with various embodiments of the present disclosure, a moisture detecting apparatus is provided. In some embodiments, the moisture detecting apparatus comprises an input port; an output port; a housing that is configured between the input port at a first end and the output port at a second end; an adsorbent material that is configured in a flow path of an inner cavity within the housing; and an electrical port comprising a controller that is coupled to the adsorbent material via one or more pin bars, wherein the controller is configured to (i) measure, using the one or more pin bars, one or more voltages on the adsorbent material and (ii) determine an electrical property of the adsorbent material based on the one or more voltages.

In some embodiments, the electrical property comprises impedance, resistance, or capacitance. In some embodiments, the one or more pin bars are embedded within the adsorbent material. In some embodiments, the one or more pin bars are configured in contact with a surface of the adsorbent material. In some embodiments, the one or more pin bars comprise a first set of pins that are configured to receive the one or more voltages. In some embodiments, the one or more pin bars comprise a second set of pins that are configured to apply an electrical current to the adsorbent material. In some embodiments, the adsorbent material comprises a plurality of pores that adsorb water. In some embodiments, the adsorbent material comprises a molecular sieve. In some embodiments, the molecular sieve comprises zeolite material. In some embodiments, the adsorbent material is configured at an offset to a centerline of the flow path.

According to another embodiment, a moisture detecting apparatus comprises an input port; an output port; a housing comprising a filter core that is configured within the housing, wherein the housing is configured to receive a fluid from the input port that passes around or through the filter core to the output port; and a connector hub that comprises one or more electrical connectors that are coupled to the filter core via one or more electrode strips that are configured on a surface of the filter core.

In some embodiments, the filter core comprises a filter drier that is adsorbent of moisture. In some embodiments, the filter core comprises aluminum silicate or zeolite materials. In some embodiments, the filter core comprises activated alumina. In some embodiments, a given electrode strip of the one or more electrode strips comprises a carrier strip that includes a plurality of electrodes; and a plurality of conductive trace/path lines that couple the plurality of electrodes to the one or more electrical connectors. In some embodiments, a given electrode strip of the one or more electrode strips comprises an outer electrode that is configured to induce an electrical current; and one or more inner electrodes that are configured to receive respectively corresponding one or more voltages. In some embodiments, the respectively corresponding one or more voltages comprise a downstream voltage that is associated with a downstream location of the filter core; and an upstream voltage that is associated with an upstream location of the filter core. In some embodiments, the one or more electrode strips comprises a first electrode strip that is configured on a first portion of the surface that is adjacent to the input port; and a second electrode strip that is configured on a second portion of the surface that is adjacent to the output port.

According to another embodiment, a method is provided. In some embodiments, the method comprises measuring, by a controller, one or more voltages of an adsorbent material that is configured in a fluid system, wherein the one or more voltages are (i) caused by inducting a current on the adsorbent material and (ii) received via one or more pins that are in contact with the adsorbent material; determining, by the controller, one or more electrical property values based on the one or more voltages; determining, by the controller, a presence of moisture in the adsorbent material based on the one or more electrical property values; determining, by the controller, a net change ratio based on the one or more electrical property values and an initial electrical impedance or resistance value that is associated with the adsorbent material in a complete dry state; and generating, by the controller, a status message based on the net change ratio.

In some embodiments, the method further comprises determining the net change ratio is greater than at least one threshold of one or more thresholds; and determining a status of the adsorbent material that is associated with the at least one threshold.

The foregoing illustrative summary, as well as other exemplary objectives and/or advantages of the disclosure, and the manner in which the same are accomplished, are further explained in the following detailed description and its accompanying drawings.

### BRIEF DESCRIPTION OF THE DRAWINGS

The description of the illustrative embodiments may be read in conjunction with the accompanying figures. It will be appreciated that, for simplicity and clarity of illustration, elements illustrated in the figures have not necessarily been drawn to scale, unless described otherwise. For example, the dimensions of some of the elements may be exaggerated relative to other elements, unless described otherwise. Embodiments incorporating teachings of the present disclosure are shown and described with respect to the figures presented herein, in which:
FIG. 1 depicts an example moisture detecting apparatus in accordance with some embodiments of the present disclosure;
FIG. 2 is a cross-sectional view of at least a portion of an example moisture detecting apparatus in accordance with some embodiments of the present disclosure;
FIG. 3 is a zoomed-in view of an example molecular sieve in accordance with some embodiments of the present disclosure;
FIG. 4 is a zoomed-in view of an example molecular sieve in accordance with some embodiments of the present disclosure;
FIG. 5 depicts an example probe configuration in accordance with some embodiments of the present disclosure;
FIG. 6 depicts an example moisture detecting apparatus in accordance with some embodiments of the present disclosure;
FIG. 7A is a top view of an example electrode strip in accordance with some embodiments of the present disclosure;
FIG. 7B is a side view of an example electrode strip in accordance with some embodiments of the present disclosure;
FIG. 8 depicts an example electrode configuration in accordance with some embodiments of the present disclosure;
FIG. 9 is a flowchart diagram of an example process for detecting moisture saturation of an adsorbent material within a fluid system in accordance with some embodiments of the present disclosure; and
FIG. 10 is a flowchart diagram of an example process for monitoring moisture saturation of an adsorbent material within a fluid system in accordance with some embodiments of the present disclosure.

### DETAILED DESCRIPTION

Some embodiments of the present disclosure will now be described more fully hereinafter with reference to the accompanying drawings, in which some, but not all embodiments of the disclosure are shown. Indeed, these disclosures may be embodied in many different forms and should not be construed as limited to the embodiments set forth herein; rather, these embodiments are provided so that this disclosure will satisfy applicable legal requirements. Like numbers refer to like elements throughout.

As used herein, terms such as "front," "rear," "top," etc., are used for explanatory purposes in the examples provided below to describe the relative position of certain components or portions of components. Furthermore, as would be evident to one of ordinary skill in the art in light of the present disclosure, the terms "substantially" and "approximately" indicate that the referenced element or associated description is accurate to within applicable engineering tolerances.

As used herein, the term "comprising" means including but not limited to and should be interpreted in the manner it is typically used in the patent context. Use of broader terms such as comprises, includes, and having should be understood to provide support for narrower terms such as consisting of, consisting essentially of, and comprised substantially of.

The phrases "in one embodiment," "according to one embodiment," and the like generally mean that the particular feature, structure, or characteristic following the phrase may be included in at least one embodiment of the present disclosure and may be included in more than one embodiment of the present disclosure (importantly, such phrases do not necessarily refer to the same embodiment).

The word "example" or "exemplary" is used herein to mean "serving as an example, instance, or illustration." Any implementation described herein as "exemplary" is not necessarily to be construed as preferred or advantageous over other implementations.

If the specification states a component or feature "may," "can," "could," "should," "would," "preferably," "possibly," "typically," "optionally," "for example," "often," or "might" (or other such language) be included or have a characteristic, that a specific component or feature is not required to be included or to have the characteristic. Such a component or feature may be optionally included in some embodiments, or it may be excluded.

As described above, there are many technical challenges and difficulties associated with using conventional sensors to detect moisture (e.g., water) in fluids. Typical moisture levels that are of interest across various applications may be in the range of 20 to 2000 ppm. Each type of application fluid (refrigerants, hydraulics fluids, lubricant oils, etc.) may comprise certain solubility of water/moisture at a given temperature and pressure. Moisture may be present either in vapor form or liquid form depending on fluid operating temperature and pressure. Conventional polymer-based sensors (e.g., ambient humidity sensors) that operate based on difference of partial pressure of moisture in air that is adsorbed to reach ambient equilibrium, may not function in liquid fluids. That is, moisture that is present in a vapor phase may saturate a polymer-based sensor and provide erroneous moisture level measurements. Similarly, moisture that is present in liquid form may be adsorbed by a polymer-based sensor and cause the sensor to provide inaccurate measurements of moisture level.

Various example embodiments of the present disclosure overcome such technical challenges and difficulties in polymer-based humidity sensors and provide various technical advancements and improvements. In accordance with various embodiments of the present disclosure, components of example moisture sensor components for determining moisture in fluids are disclosed. In some embodiments, electrical property changes of an adsorbent material, such as a molecular sieve or a filter drier core, may be leveraged to measure/detect moisture in liquids/oils, fuels, gases and process fluids (e.g., bi-polar).

In some embodiments, a moisture sensor comprises a molecular sieve that is configured within an inner cavity of a metal housing. In some embodiments, the moisture sensor further comprises one or more pin bars embedded within the molecular sieve to determine an electrical property (e.g., impedance, resistance, and/or capacitance) of the molecular sieve, which may be representative of a presence or amount of moisture in a fluid, such as refrigerant. In some embodiments, the moisture sensor comprises a molecular sieve with two sets of metal pin bars that are embedded within the molecular sieve to form electrical leads to determine a change in electrical property (e.g., resulting from a presence of moisture in a fluid). In some embodiments, the molecular sieve may comprise a shape, such as cylindrical or toroid, that reduces flow resistance of a fluid passing along the molecular sieve. In some embodiments, the molecular sieve is configured at an offset of a centerline of fluid flow and at a widened portion of the metal housing to increase interaction of the fluid with the molecular sieve.

In some embodiments, the moisture sensor comprises a controller, such as an application-specific integrated circuit (ASIC), a microcontroller, or one or more processors, that is embedded within an electrical port portion of the metal enclosure. In some embodiments, the controller is configured to receive electrical property values of a molecular sieve and generate a signal (e.g., analog/digital) based on the electrical property values that may be received by a main controller via a connection to the electrical port for alarm/status monitoring. In some embodiments, when the molecular sieve is in a dry state, an electrical property value that is determined by using the one or more pin bars may comprise an initial value within a range. In some embodiments, the molecular sieve may comprise a molecular sieve that comprises certain adsorption properties, such as being capable of adsorbing certain molecules but not others. For example, a molecular sieve that is configured for monitoring moisture in a refrigerant may not adsorb the refrigerant (e.g., electrical property remains near or at the initial value) but may adsorb moisture, such as water, if present in the refrigerant. In some embodiments, adsorption of moisture by the molecular sieve may cause a change in electrical property (e.g., a decrease in impedance, resistance, and/or capacitance) of the molecular sieve.

In some alternative embodiments, moisture adsorption may be measured based on surface resistivity of a molecular sieve. In some embodiments, surface resistivity of a molecular sieve may be determined by using a pre-loaded spring including a 4-point probe that comprises two outer pins that induce current and two inner pins that measure voltage. As such, moisture of the molecular sieve may be determined based on a relationship between voltage to moisture levels. In some embodiments, a molecular sieve reaching adsorption capacity by weight may indicate a maximum range of moisture detected in a fluid. In some embodiments, an electrical property of the molecular sieve comprising a lowest relative value (e.g., impedance and/or resistance) within a range may provide an indication of saturation. In some embodiments, a moisture measurement range of a molecular sieve may be configured by increasing or decreasing molecular sieve weight.

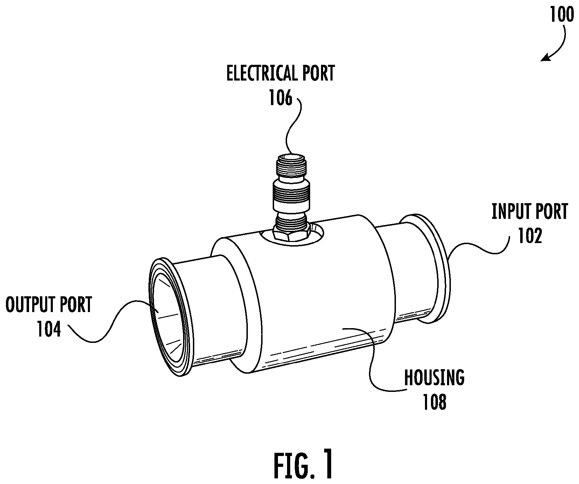

FIG. 1 depicts an example moisture detecting apparatus 100 in accordance with some embodiments of the present disclosure. The moisture detecting apparatus 100 comprises a housing 108 that includes an input port 102, an output port 104, and an electrical port 106. In some embodiments, the input port 102 and the output port 104 may comprise threaded or brazed connections with fluid lines or hoses. In some embodiments, the moisture detecting apparatus 100 may be configured as an inline sensor between refrigerant lines such that refrigerant may be received into an inner cavity of the housing 108 via the input port 102 and transported out of the inner cavity of the housing 108 via the output port 104. In some embodiments, the housing 108 comprises an enclosure that is sufficient to withstand or handle line pressure of a target application. For example, the housing 108 may comprise a metal housing that is capable of withstanding air-conditioning working pressures. In some example embodiments, the housing 108 comprises copper, aluminum, brass, or steel tubing. In some embodiments, the electrical port 106 comprises a controller that is coupled (e.g., via a sealed wire feed) to a set of probes interfaced with a moisture gathering element configured within the inner cavity of the housing 108. In some embodiments, the controller may comprise a processing element, such as an ASIC or a microcontroller, that is configured to determine an amount of moisture in a fluid that flows through the inner cavity of the housing 108.

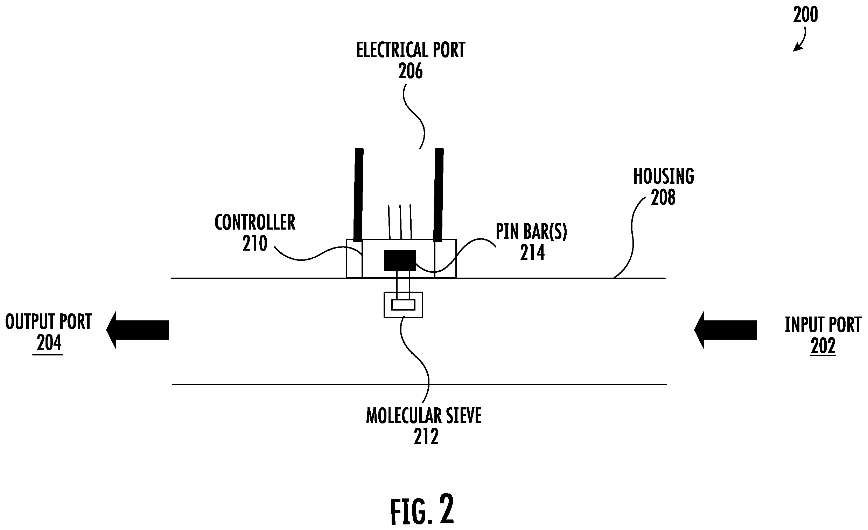

FIG. 2 is a cross-sectional view of at least a portion of an example moisture detecting apparatus 200 in accordance with some embodiments of the present disclosure. The moisture detecting apparatus 200 is an example of the moisture detecting apparatus 100 of FIG. 1. The moisture detecting apparatus 200 comprises a housing 208 that is configured between an input port 202 at a first end and an output port 204 at a second end. The housing 208 comprises a tubular shape and includes an inner cavity that comprises a flow path in which fluid may pass through from the input port 202 to the output port 204. The housing 208 further comprises a molecular sieve 212 (or an adsorbent material) that is configured in the flow path of the inner cavity of the housing 208. In some embodiments, the molecular sieve 212 is configured such that any moisture in a fluid that passes through the inner cavity of the housing 208 may be adsorbed by the molecular sieve 212. In some embodiments, the moisture detecting apparatus 200 is configured to detect moisture (e.g., water) in fluids by selective adsorption of moisture using the molecular sieve 212. Adsorption of moisture by the molecular sieve 212 may cause a change in an electrical property (e.g., impedance, resistance and/or capacitance) of the molecular sieve 212 that may be determined by measuring voltages on the molecular sieve 212 via one or more pin bars 214.

In some embodiments, the molecular sieve 212 is configured as a moisture gathering element that may be used to determine accumulated estimation of net moisture in a monitored fluid (e.g., refrigerant). In some embodiments, the molecular sieve 212 comprises zeolite material, such as crystalline metal aluminosilicates that have a three-dimensional interconnecting network of silica and alumina tetrahedra structures. In some embodiments, the molecular sieve 212 may comprise pores that adsorb moisture in the form of molecules that are of a particular size. In some embodiments, the molecular sieve 212 may comprise a pore size that is within a range from 3 to 12 angstroms (A). As such, a molecule that is smaller than the pore size of the molecular sieve 212 may be adsorbed by Van der Waals forces into the molecular sieve 212. For example, water molecules may be less than the pore size of molecular sieve 212 (e.g., less than 3A) and may be adsorbed into the molecular sieve 212.

In some embodiments, the molecular sieve 212 may be sized to a desired amount of adsorption by parts per million (ppm). For example, the molecular sieve 212 may comprise a configurable cylindrical molecular sieve length that is adjusted for a given range of moisture level detection in a given volume of fluid. In some embodiments, the molecular sieve 212 may be cut to length based on a desired moisture saturation level.

In some embodiments, to further increase interaction of a fluid with the molecular sieve 212, the molecular sieve 212 may be configured at an offset to the centerline of flow. In some embodiments, the molecular sieve 212 may comprise a design that minimizes flow resistance when configured in a path of a fluid. In some embodiments, the molecular sieve 212 may comprise a cylindrical, toroid, or any other shape that may minimize flow resistance. In some embodiments, the housing 208 may comprise a widened portion where the molecular sieve 212 may be configured to further reduce flow resistance and minimize pressure drop of a fluid passing through the inner cavity of the housing 208.

The presence of molecules adsorbed into the molecular sieve 212 may be representative of a moisture level of a fluid that has passed through the housing 208. In particular, an electrical property of the molecular sieve 212 may change as a quantity of molecules adsorbed into the molecular sieve 212 increases. For example, impedance and/or resistance of the molecular sieve 212 may decrease as more molecules (e.g., moisture) are adsorbed into the molecular sieve 212. Similarly, the capacitance of the molecular sieve 212 may increase with increased molecule adsorption. As such, a moisture level of a fluid may be determined based on an electrical property of the molecular sieve 212.

The housing 208 is further coupled to an electrical port 206. The electrical port 206 comprises a controller 210 and one or more pin bars 214 that are coupled to the controller 210. In some embodiments, the electrical port 206 may comprise an electrical port sub-housing, such as a sealed glass feed-through, that comprises the controller 210 configured therein. In some embodiments, the controller 210 may comprise an ASIC or a microcontroller. In some embodiments, the one or more pin bars 214 may comprise electrically conductive leads that are configured to provide an interface between the controller 210 and the molecular sieve 212. In some embodiments, at least a portion of the one or more pin bars 214 is embedded within the molecular sieve 212. In some embodiments, the controller 210 may apply an electrical alternating current (AC) voltage to the molecular sieve 212 (e.g., via a first set of pins of the one or more pin bars 214) to generate voltages that may be measured from the molecular sieve 212 (e.g., via a second set of pins of the one or more pin bars 214) to determine an electrical property of the molecular sieve 212, and in turn, a moisture content/level of the molecular sieve 212.

In some embodiments, the controller 210 may be configured to process and/or determine electrical property values of the molecular sieve 212 based on changes (e.g., adsorption of moisture) to the molecular sieve 212. The one or more pin bars 214 may provide an interface between the controller 210 and the molecular sieve 212 for transmitting signals that are representative of electrical property values from the molecular sieve 212 to the controller 210. In some embodiments, a quantity of the one or more pin bars 214 may vary based on a desired sensitivity or accuracy (e.g., more pin bars may provide increased sensitivity or accuracy). In some example embodiments, the controller 210 may further generate moisture values or status signals based on the electrical property values of the molecular sieve 212 and provide the moisture values or status signals to a master controller for a control action, alarm, or status monitoring.

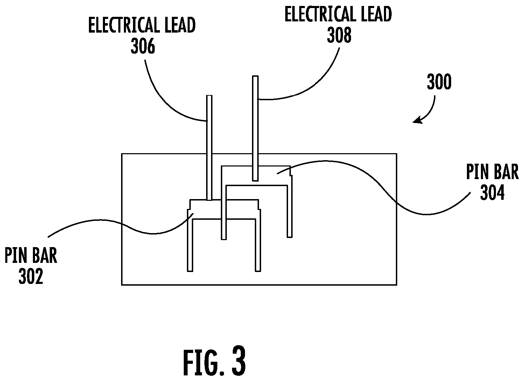

FIG. 3 is a zoomed-in view of an example molecular sieve 300 in accordance with some embodiments of the present disclosure. The molecular sieve 300 is an example of the molecular sieve 212 of FIG. 2. As depicted in FIG. 3, the pin bar 302 and the pin bar 304 may each comprise a set (e.g., two or more) of prongs or probes that are embedded within the molecular sieve 300. The pin bar 302 and the pin bar 304 may be coupled to a controller (e.g., microcontroller or ASIC) via the electrical lead 306 and the electrical lead 308, respectively, to receive electrical property values from the molecular sieve 300. In some embodiments, the electrical lead 306 and the electrical lead 308 may be coupled to a controller via a sealed wire feed through an electrical port. In some embodiments, the molecular sieve 300 may comprise a shape or profile.

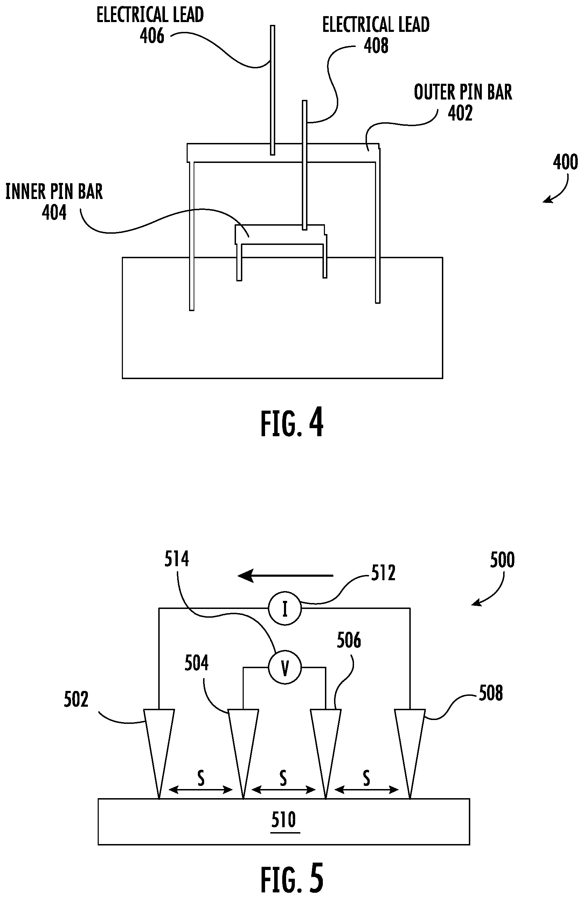

FIG. 4 is a zoomed-in view of an example molecular sieve 400 in accordance with some embodiments of the present disclosure. The molecular sieve 400 is another example of the molecular sieve 212 of FIG. 2. The outer pin bar 402 and the inner pin bar 404 may each comprise a set (e.g., two or more) of prongs or probes that are configured in contact with a surface (e.g., via pre-loaded springs) along a length of the molecular sieve 400. The outer pin bar 402 and the inner pin bar 404 may be coupled to a controller (e.g., microcontroller or ASIC) via the electrical lead 406 and the electrical lead 408, respectively, to determine surface/sheet resistivity of the molecular sieve 400 using a 4-point probe method, which is described in further detail with respect to the description of FIG. 5.

FIG. 5 depicts an example probe configuration 500 in accordance with some embodiments of the present disclosure. In some embodiments, the probe configuration 500 may be used to determine moisture adsorption of a fluid system 510 (e.g., molecular sieve) based on surface/sheet resistivity. The probe configuration 500 comprises (i) a pair of outer pins 502, 508 and (ii) a pair of inner pins 504, 506 that are configured between the pair of outer pins 502, 508. The outer pin 502 is configured adjacent to and a distance s from the inner pin 504, the inner pin 504 is configured adjacent to and a distance s from the inner pin 506, and the inner pin 506 is configured adjacent to and a distance s from the outer pin 508. In some embodiments, the outer pins 502, 508 and the inner pins 504, 506 may be configured with a pre-loaded spring as part of a sensor enclosure that exerts the outer pins 502, 508 and the inner pins 504, 506 with sufficient contact force against a surface of the fluid system 510. By using a controller (e.g., microcontroller or ASIC) that is coupled to the outer pins 502, 508 and the inner pins 504, 506, an electrical current 512 may be induced at the outer pins 502, 508 to provide voltage 514 that is measurable from the inner pins 504, 506. The voltage 514 may be used to determine moisture adsorption of the fluid system 510 based on a relationship between voltage and moisture levels.

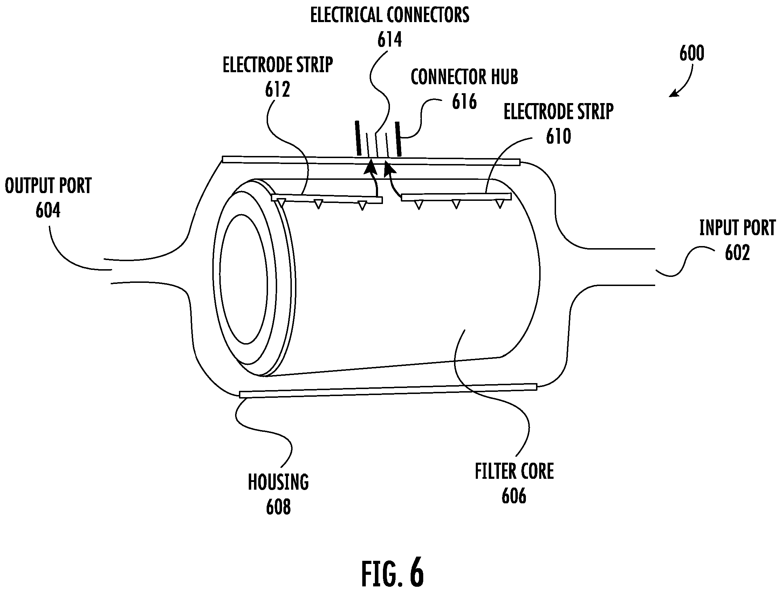

FIG. 6 depicts an example moisture detecting apparatus 600 in accordance with some embodiments of the present disclosure. The moisture detecting apparatus 600 comprises a housing 608 that includes a filter core 606 configured therein. In some embodiments, the housing 608 is configured to receive a fluid from an input port 602 such that a flow path is provided by entering and passing around and/or through the filter core 606 to an output port 604. According to various embodiments of the present disclosure, the moisture detecting apparatus 600 may comprise a filtration device that is configured to adsorb moisture from a fluid system. By passing a fluid around and/or through the filter core 606, moisture (e.g., water molecules) in the fluid may be adsorbed by the filter core 606.

In some embodiments, the filter core 606 comprises pores that adsorb moisture in the form of molecules that are of a particular size. In some embodiments, the filter core 606 comprises materials that have a pore size that is within a range from 3A to 12A. Molecules that are smaller than the pore size of the filter core 606 material may be adsorbed. For example, water molecules may be less than 3A and may be adsorbed by the filter core 606. As such, the filter core 606 may filter out water to dehydrate materials across different applications. In some example embodiments, the filter core 606 comprises a filter drier that adsorbs water from refrigerant in a refrigerant circuit. In some embodiments, the filter core 606 comprises aluminum silicate or zeolite materials, which may comprise crystalline molecules that include three-dimensional interconnecting networks of silica and alumina tetrahedra structures. In some embodiments, the filter core 606 comprises activated alumina (e.g., Al₂O₃).

In some embodiments, the filter core 606 may comprise a volume that is capable of providing a given amount of adsorption capacity. For example, the filter core 606 may comprise a moisture adsorption capacity that is within a range of approximately 12% to 22% by weight. In some embodiments, surface/sheet resistivity of the filter core 606 may be determined to assess moisture saturation of filter core 606. As moisture or molecules are adsorbed by the filter core 606, the accumulation of moisture or molecules in the pores of the material of the filter core 606 may alter surface/sheet resistivity of the filter core 606. For example, as the filter core 606 is saturated with moisture or molecules, the surface/sheet resistance of the filter core 606 may decrease. Accordingly, a "dry" or unsaturated filter core 606 may comprise an electrical resistance value that is relatively higher than an electrical resistance of a saturated filter core 606.

In some embodiments, surface/sheet resistivity of the filter core 606 may be obtained by determining electrical resistance via the electrode strip 610 and the electrode strip 612. As depicted in FIG. 6, the electrode strip 610 is configured on a surface of a first portion of the filter core 606 that is adjacent to the input port 602 and the electrode strip 612 is configured on surface of a second portion of the filter core 606 that is adjacent to the output port 604. Such configuration of the electrode strip 610 and the electrode strip 612 may minimize surface area obstruction of the filter core 606 as well as provide ease of assembly during manufacturing. In some embodiments, a controller (e.g., microcontroller or ASIC) may be coupled to the electrical connectors 614 via the connector hub 616 to send and/or receive electrical signals to the electrode strip 610 and the electrode strip 612. In some embodiments, a controller may be configured to (i) apply current to the electrode strip 610 and the electrode strip 612, (ii) measure voltages from the electrode strip 610 and the electrode strip 612, and (iii) generate and/or communicate alerts to a master controller for further actions. In some embodiments, the connector hub 616 may comprise a sealed feed-through connector assembly for extending the electrical connectors 614 to the electrode strip 610 and the electrode strip 612. The connector hub 616 may be capable of withstanding working pressures and temperatures of various applications suitable for the moisture detecting apparatus 600.

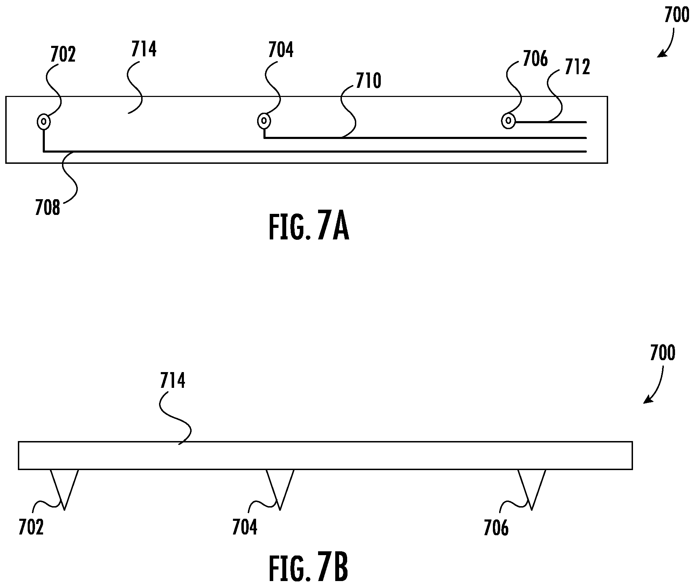

FIG. 7A is a top view of an example electrode strip 700 in accordance with some embodiments of the present disclosure. The electrode strip 700 is an example of the electrode strip 610 or the electrode strip 612 of FIG. 6. The electrode strip 700 comprises a flexible carrier strip 714 including electrodes 702, 704, and 706 that are embedded therein. The electrodes 702, 704, and 706 may be spaced at regular intervals. In some embodiments, the intervals may be in proportion (e.g., a fraction, such as one-third) to a thickness of a filter core the electrodes 702, 704, and 706 are configured with. In some embodiments, the flexible carrier strip may comprise a polyimide film that is made by heating a polyimide resin. The electrode strip 700 further comprises conductive trace/path lines 708, 710, and 712 that couple the electrodes 702, 704, and 706 to electrical connectors (e.g., electrical connectors 614), respectively. In some embodiments, the electrodes 702, 704, and 706 may be spaced a predetermined distance from each other, such as D/3, wherein D is representative of a diameter of a filter core (e.g., filter core 606).

As depicted in FIG. 7A, the conductive trace/path line 708 comprises the longest length relative to the conductive trace/path lines 710 and 712. As such, electrode 702 may be a furthest distance from an electrical connector (and in some embodiments, a center of a filter core) and configured as an outermost electrode relative to the inner electrodes 704 and 706. In some example embodiments, a current may be induced on a filter core at the outermost electrode 702 and resulting voltages may be measured from the filter core at the inner electrodes 704 and 706 to determine surface/sheet resistivity.

FIG. 7B is a side view of an example electrode strip 700 in accordance with some embodiments of the present disclosure. The electrodes 702, 704, and 706 comprise pointed ends that may be used as electrical probes, such as of a filter core (e.g., filter core 606) surface. In some embodiments, the electrodes 702, 704, and 706 may be used to determine surface/sheet resistivity of a filter core based on a configuration that is similar to the description with respect to FIG. 5. For example, referring back to FIG. 6, a current may be induced at (i) an outer electrode of electrode strip 610 that is nearest to the input port 602 and (ii) an outer electrode of electrode strip 612 that is nearest to the output port 604. Voltage resulting from the induced current may be received and measured at (i) inner electrodes of the electrode strip 610 and (ii) inner electrodes of the electrode strip 612. In some embodiments, a geometrical correction factor may be determined to adapt surface/sheet resistances for a range of moisture saturation levels based on filter core thickness and spacing between electrodes.

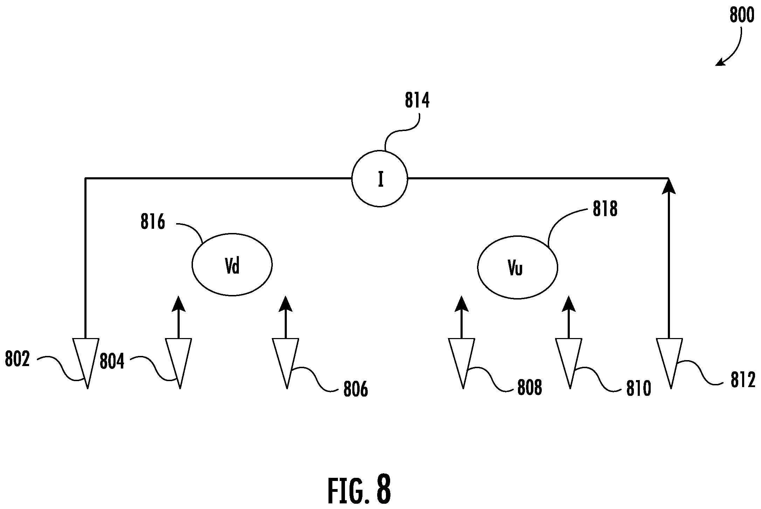

FIG. 8 depicts an example electrode configuration 800 in accordance with some embodiments of the present disclosure. In some embodiments, the electrode configuration 800 may be used to determine moisture saturation of an adsorbent material, such as a filter core (e.g., filter core 606) or a molecular sieve (e.g., molecular sieve 212), based on surface/sheet resistivity. The electrode configuration 800 may take into account sheet resistance contribution of fluid (e.g., refrigerant) flowing above an adsorbent material surface. The electrode configuration 800 comprises (i) outer electrodes 802, 812 and (ii) inner electrodes 804, 806, 808, and 810 that are configured between the outer electrodes 802, 812. In some embodiments, the outer electrodes 802, 812 and the inner electrodes 804, 806, 808, and 810 may be provided by two or more electrode strips. In some example embodiments, a first electrode strip may comprise the outer electrode 802 and the inner electrodes 804, 806, while a second electrode strip may comprise the outer electrode 812 and the inner electrodes 808, 810.

By using a controller that is coupled to the outer electrodes 802, 812 and the inner electrodes 804, 806, 808, and 810, an electrical current 814 may be induced at the outer electrodes 802, 812 to provide (i) a downstream voltage *V_{d}* 816 that is measurable from inner electrodes 804, 806 and (ii) an upstream voltage *Vᵤ* 818 that is measurable from inner electrodes 808, 810. The voltage *V_{d}* 816 and the voltage *Vᵤ* 818 may be used to determine surface/sheet resistivity of an adsorbent material. In some embodiments, the downstream voltage *V_{d}* 816 may be representative of voltage measured at a downstream location (e.g., adjacent to output port 604) of an adsorbent material and the upstream voltage *Vᵤ* 818 may be representative of voltage measured at an upstream location (e.g., adjacent to input port 602) of the adsorbent material.

According to various embodiments of the present disclosure, moisture saturation and/or status of an adsorbent material may be determined based on its surface/sheet resistivity. In some embodiments, changes in surface saturation in the direction of refrigerant flow along a length of an adsorbent material may be determined based on a gradient of surface/sheet resistivity along the adsorbent material length in response to accumulated moisture saturation over time. In some embodiments, the downstream voltage *V_{d}* 816 and the upstream voltage *Vᵤ* 818 may be used to determine whether an adsorbent material is dry (e.g., dry state), has accumulated moisture (e.g., water) across a length of the adsorbent material, or is saturated. In some embodiments, a time to saturate may also be predicted based on rate (e.g., gradient) of moisture accumulation from continuous monitoring of surface/sheet resistivity gradient over time.

According to various embodiments of the present disclosure, an adsorbent material, such as a molecular sieve or a filter drier core, may be configurable (e.g., sized by weight or dimensions) to suit a desired moisture adsorption capability in a fluid system before fully saturating (or a desired range of electrical property or surface/sheet resistance value changes). For example, a refrigeration system comprising a volume of 1 liter refrigerant and a moisture sensor that is configured to assess moisture level of 100 ppm, may be configured with a molecular sieve volume that is designed such that the molecular sieve may be fully saturated (over time) with 100 ppm and comprise a lowest impedance or resistance value (or highest capacitance value) when at 100 ppm. In another example, a refrigeration comprising a moisture sensor that is configured to monitor 200 ppm for 1 liter of refrigerant may be designed such that its impedance or resistance value is lowest (or capacitance value is highest) when at 200 ppm. To suit application specification, adsorbent material volume may be configured either at field or at factory by adjusting, for example, a length of an adsorbent material by a field engineer.

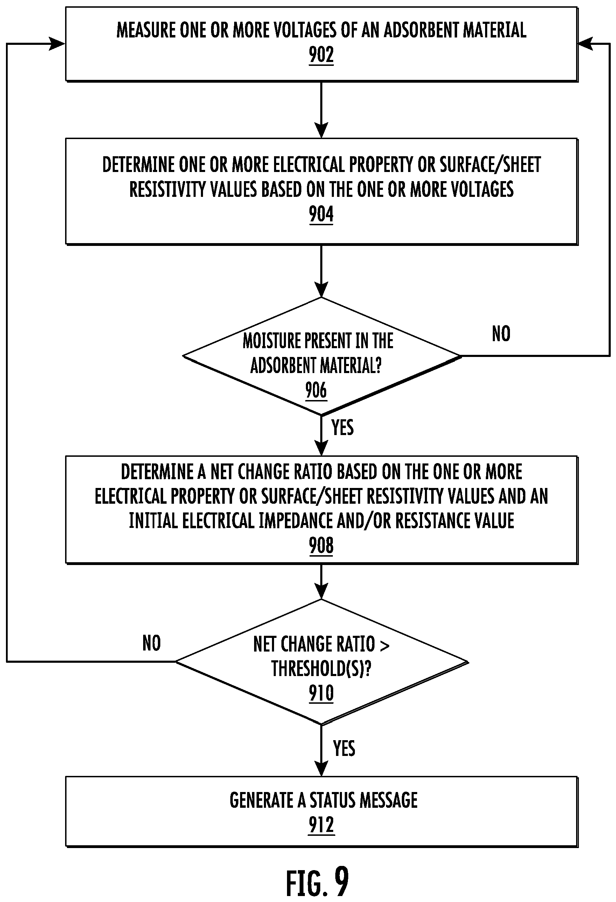

FIG. 9 is a flowchart diagram of an example process 900 for detecting moisture saturation of an adsorbent material within a fluid system in accordance with some embodiments of the present disclosure. In some embodiments, via the various steps/operations of the process 900, a controller determines a rate at which an adsorbent material, such as a molecular sieve or a filter drier core, that is configured in a fluid system, such as a refrigerant system, is saturating and generates a proactive warning of saturation.

In some embodiments, the process 900 begins at step/operation 902 when a controller measures one or more voltages of an adsorbent material. The adsorbent material may comprise a molecular sieve or a filter core that is configured in a fluid system to adsorb moisture (e.g., water) from fluid (e.g., refrigerant) circulating in the fluid system. The one or more voltages may be received by the controller via pins, probes, or electrodes that are in contact (either on the surface or below the surface) with the adsorbent material. In some example embodiments, the one or more voltages may be caused by inducing a current on the adsorbent material via a first set of pins that are configured at outer portions of the adsorbent material and the one or more voltages may be measured using a second set of pins that are configured at inner portions of the adsorbent material between the first set of pins.

In some embodiments, subsequent to step/operation 902, the example process proceeds to step/operation 904, where the controller determines one or more electrical property (e.g., impedance, resistance, and/or capacitance) or surface/sheet resistivity values based on the one or more voltages. For example, Ohm's law may be used to determine one or more resistance values given the induced current and the measured one or more voltage values.

In some embodiments, subsequent to step/operation 904, the example process proceeds to step/operation 906, where the controller determines the presence of moisture in the adsorbent material based on the one or more electrical property or surface/sheet resistivity values. In some embodiments, an initial electrical impedance and/or resistance value that is associated with the adsorbent material in a complete dry state may comprise a highest value (or lowest capacitance value) within a range. The complete dry state may be representative of a fluid system comprising moistureless (e.g., waterless) fluid circulating therein and across or through the adsorbent material. In some embodiments, if the one or more electrical property or surface/sheet resistivity values are equal to the initial electrical impedance and/or resistance value, the controller may determine that no moisture is present in the adsorbent material. In some embodiments, if the controller determines that no moisture is present in the adsorbent material, subsequent to step/operation 906, the example process proceeds to step/operation 902, where the controller continues to measure one or more voltages of the adsorbent material.

In some embodiments, if the one or more electrical property or surface/sheet resistivity values are not equal to the initial electrical impedance and/or resistance value, the controller may determine that moisture is present. In some embodiments, moisture in a fluid may be adsorbed into the adsorbent material (e.g., no longer in a complete dry state) and change the electrical impedance and/or resistance value of the adsorbent material to a lower value (or to a higher capacitance value) within a range. For example, electrical resistance of an adsorbent material may decrease as the pores of the adsorbent material are filled with water molecules.

In some embodiments, if the controller determines that moisture is present in the adsorbent material, subsequent to step/operation 906, the example process proceeds to step/operation 908, where the controller determines a net change ratio based on the one or more electrical property or surface/sheet resistivity values and an initial electrical impedance and/or resistance value that is associated with the adsorbent material in a complete dry state. In some embodiments, the net change ratio may be representative of a saturation status or moisture level. In some embodiments, the net change ratio may represent a rate at which the electrical property or surface/sheet resistivity values of the adsorbent material are changing due to adsorption of moisture in the fluid system to predict full saturation of the adsorbent material. In some embodiments, the net change ratio comprises a comparison of one or more instantaneous electrical property or surface/sheet resistivity values and an initial (e.g., complete dry state) electrical property or surface/sheet resistivity value.

In some embodiments, the net change ratio may be determined based on a quotient of the one or more electrical property or surface/sheet resistivity values (e.g., determined from step/operation 904) over the initial electrical impedance and/or resistance value that is associated with the adsorbent material in a complete dry state. In some embodiments, the net change ratio may comprise a change of electrical property of surface/sheet resistivity in time with respect to an initial electrical impedance and/or resistance value of the adsorbent material when in a complete dry state. In some embodiments, a time-averaged electrical property of surface/sheet resistivity values over a time window may be determined to accommodate momentary fluctuations or reduce noise.

In some embodiments, subsequent to step/operation 908, the example process proceeds to step/operation 910, where the controller determines whether the net change ratio is greater than one or more thresholds. The net change ratio may be compared to one or more preset thresholds as per application to determine moisture levels present in fluid systems or provide relative measurement or status signals. In some embodiments, the one or more thresholds may be associated with a status of the adsorbent material (e.g., complete dry, wet (or presence of moisture), or saturated).

In some embodiments, if the net change ratio is not greater than one or more thresholds, subsequent to step/operation 910, the example process proceeds to step/operation 902, where the controller continues to measure one or more voltages of the adsorbent material.

In some embodiments, if the net change ratio is greater than one or more thresholds, subsequent to step/operation 910, example process proceeds to step/operation 912, where the controller generates a status message based on the one or more thresholds exceeded by the net change ratio. In some embodiments, generating the status message further comprises determining a status of the adsorbent material (e.g., complete dry, wet (or presence of moisture), or saturated) based on the one or more thresholds exceeded by the net change ratio. In some embodiments, the status message may be used to communicate, to a master controller, a status of the adsorbent material based on the one or more thresholds exceeded by the net change ratio.

In some embodiments, early warning alerts of moisture levels or saturation may be generated by a controller. Such early warning may be useful to plan maintenance of fluid systems. In some embodiments, a moisture saturation switch may be provided where a digital ON/OFF signal may be issued by a controller when a monitored adsorbent material is saturated or comprises a given moisture level based on determined electrical property or surface/sheet resistance of the adsorbent material, rather than actual measurement of moisture levels. In some embodiments, alerts as disclosed herewith may be used to determine control actions that may be performed by maintenance personnel. Accordingly, a controller may be configured to generate proactive alerts to initiate need-based maintenance rather than, or in addition to, scheduled maintenance or attending to symptoms/faults.

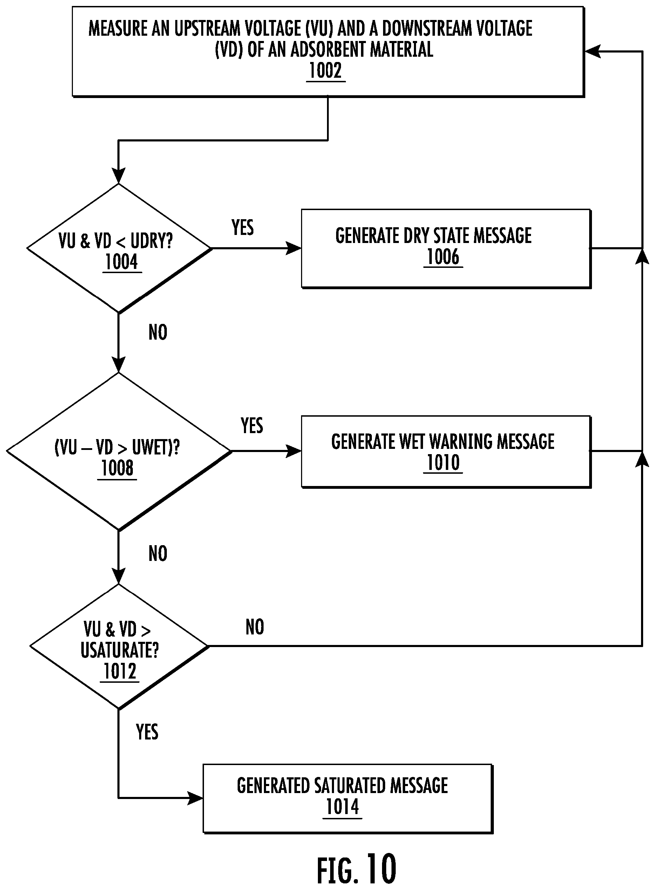

FIG. 10 is a flowchart diagram of an example process 1000 for monitoring moisture saturation of an adsorbent material within a fluid system in accordance with some embodiments of the present disclosure. In some embodiments, via the various steps/operations of the process 1000, a controller determines (i) surface/sheet resistivity of an adsorbent material, such as a molecular sieve or a filter drier core and (ii) status alerts based on the surface/sheet resistivity.

In some embodiments, the process 1000 begins at step/operation 1002 when a controller measures an upstream voltage *Vu* and a downstream voltage *Vd* of an adsorbent material. Measurements of the upstream voltage *Vu* and the downstream voltage *Vd* may be obtained by sampling voltage readings at specific locations on the adsorbent material. In some embodiments, the upstream voltage *Vu* may be associated with an upstream location of the adsorbent material, such as a location on the adsorbent material that is adjacent to an input port. In some embodiments, the downstream voltage *Vd* may be associated with a downstream location of the adsorbent material, such as a location on the adsorbent material that is adjacent to an output port. In some embodiments, the upstream voltage *Vu* and the downstream voltage *Vd* may be generated by inducing a current on the adsorbent material. In some embodiments, electrical resistance or impedance of the adsorbent material may change based on an amount of moisture contained in the adsorbent material. Accordingly, the upstream voltage *Vu* and the downstream voltage *Vd* may represent an amount of moisture in the adsorbent material where the upstream voltage *Vu* and a downstream voltage *Vd* are measured given a known electrical current that is induced on the adsorbent material.

In some embodiments, subsequent to step/operation 1002, the example process proceeds to step/operation 1004, where the controller determines whether a moisture level based on *Vu* and *Vd* is less than a threshold *Udry.* The threshold *Udry* may be representative of a maximum moisture level that is associated with a dry state for a given type of fluid.

In some embodiments, if the moisture level based on *Vu* and *Vd* is less than the threshold *Udry,* subsequent to step/operation 1004, the example process proceeds to step/operation 1006, where the controller generates a dry state message. In some embodiments, determining whether the moisture level based on *Vu* and *Vd* is less than the threshold *Udry* comprises verifying an initial dry or non-saturated state of the adsorbent material in the fluid system. In some embodiments, subsequent to step/operation 1006, the example process proceeds to step/operation 1002, where the controller continues to measure the upstream voltage *Vu* and the downstream voltage *Vd.*

In some embodiments, if the moisture level based on *Vu* and *Vd* is not less than the threshold *Udry* (i.e., the moisture level based on *Vu* and *Vd* is greater than the threshold *Udry*)*,* subsequent to step/operation 1004, the example process proceeds to step/operation 1008, where the controller determines whether a difference between *Vu* and *Vd* is greater than a threshold *Uwet.* The difference between *Vu* and *Vd* may represent an uneven adsorption of moisture by the adsorbent material during an operational lifetime of the adsorbent material, especially at upstream locations of the adsorbent material that are adjacent to an input port. As such, *Vu* may be greater than *Vd* which is representative of an upstream location of the adsorbent material that has adsorbed more moisture than a downstream location. The threshold *Uwet* may be representative of a maximum differential of moisture that is allowed between *Vu* and *Vd.* Accordingly, a condition may be monitored where *Vu* is greater than *Vd* by an amount allowable by the threshold *Uwet.*

In some embodiments, if the difference between *Vu* and *Vd* is greater than the threshold *Uwet,* subsequent to step/operation 1008, the example process proceeds to step/operation 1010, where the controller generates a wet warning message. In some embodiments, subsequent to step/operation 1010, the example process proceeds to step/operation 1002, where the controller continues to measure the upstream voltage *Vu* and the downstream voltage *Vd.*

Moisture and/or saturation may spread across the adsorbent material towards equilibrium (i.e., *Vd* equal to *Vu*) where a difference between *Vu* and *Vd* may be less than the threshold *Uwet.* In some embodiments, if the difference between *Vu* and *Vd* is not greater than the threshold *Uwet* (i.e., the difference between *Vu* and *Vd* is less than the threshold *Uwet*)*,* subsequent to step/operation 1008, the example process proceeds to step/operation 1012, where the controller determines whether the moisture level based on *Vu* and *Vd* is greater than a threshold *Usaturate.* The threshold *Usaturate* may be representative of a maximum moisture level that is associated with an adsorbent material that is significantly saturated or unable to adsorb additional moisture.

In some embodiments, determining whether the moisture level based on *Vu* and *Vd* is greater than the threshold *Usaturate* may comprise determining and/or predicting a time to significant saturation of the adsorbent material by continuously measuring surface/sheet resistance over a given period of time and assessing a rate of change of adsorption based on the continuous surface/sheet resistance measurements. In some embodiments, a regression line may be fitted to the rate of change of adsorption and a slope of the regression line may be determined such that the slope may be used to determine a time to saturation estimation for proactive maintenance scheduling.

In some embodiments, if the moisture level based on *Vu* and *Vd* is greater than the threshold *Usaturate,* subsequent to step/operation 1012, the example process proceeds to step/operation 1014, where the controller generates a saturated message.

In some embodiments, if the moisture level based on *Vu* and *Vd* is not greater than the threshold *Usaturate* (i.e., the moisture level based on *Vu* and *Vd* is less than the threshold *Usaturate*)*,* subsequent to step/operation 1012, the example process proceeds to step/operation 1002, where the controller continues to measure the upstream voltage *Vu* and the downstream voltage *Vd.*

It is to be understood that the disclosure is not to be limited to the specific embodiments disclosed, and that modifications and other embodiments are intended to be included within the scope of the appended claims. Although specific terms are employed herein, they are used in a generic and descriptive sense only and not for purposes of limitation, unless described otherwise.

## Claims

1. A moisture detecting apparatus comprising:
an input port;
an output port;
a housing that is configured between the input port at a first end and the output port at a second end;
an adsorbent material that is configured in a flow path of an inner cavity within the housing; and
an electrical port comprising a controller that is coupled to the adsorbent material via one or more pin bars, wherein the controller is configured to (i) measure, using the one or more pin bars, one or more voltages on the adsorbent material and (ii) determine an electrical property of the adsorbent material based on the one or more voltages.

2. The moisture detecting apparatus of claim 1, wherein the electrical property comprises impedance, resistance, or capacitance.

3. The moisture detecting apparatus of claim 1, wherein the one or more pin bars are (i) embedded within the adsorbent material or (ii) configured in contact with a surface of the adsorbent material.

4. The moisture detecting apparatus of claim 1, wherein the one or more pin bars comprise:
a first set of pins that are configured to receive the one or more voltages; and
a second set of pins that are configured to apply an electrical current to the adsorbent material.

5. The moisture detecting apparatus of claim 1, wherein the adsorbent material comprises a plurality of pores that adsorb water.

6. The moisture detecting apparatus of claim 1, wherein the adsorbent material comprises a molecular sieve.

7. The moisture detecting apparatus of claim 6, wherein the molecular sieve comprises zeolite material.

8. The moisture detecting apparatus of claim 1, wherein the adsorbent material is configured at an offset to a centerline of the flow path.

9. A moisture detecting apparatus comprising:
an input port;
an output port;
a housing comprising a filter core that is configured within the housing, wherein the housing is configured to receive a fluid from the input port that passes around or through the filter core to the output port; and
a connector hub that comprises one or more electrical connectors that are coupled to the filter core via one or more electrode strips that are configured on a surface of the filter core.

10. The moisture detecting apparatus of claim 9, wherein the filter core comprises a filter drier that is adsorbent of moisture.

11. The moisture detecting apparatus of claim 9, wherein the filter core comprises aluminum silicate, zeolite materials, or activated alumina.

12. The moisture detecting apparatus of claim 9, wherein a given electrode strip of the one or more electrode strips comprises:
a carrier strip that includes a plurality of electrodes; and
a plurality of conductive trace/path lines that couple the plurality of electrodes to the one or more electrical connectors.

13. The moisture detecting apparatus of claim 9, wherein a given electrode strip of the one or more electrode strips comprises:
an outer electrode that is configured to induce an electrical current; and
one or more inner electrodes that are configured to receive respectively corresponding one or more voltages, wherein the respectively corresponding one or more voltages comprise (i) a downstream voltage that is associated with a downstream location of the filter core or (ii) an upstream voltage that is associated with an upstream location of the filter core.

14. The moisture detecting apparatus of claim 9, wherein the one or more electrode strips comprises:
a first electrode strip that is configured on a first portion of the surface that is adjacent to the input port; and
a second electrode strip that is configured on a second portion of the surface that is adjacent to the output port.

15. A method comprising:
measuring, by a controller, one or more voltages of an adsorbent material that is configured in a fluid system, wherein the one or more voltages are (i) caused by inducting a current on the adsorbent material and (ii) received via one or more pins that are in contact with the adsorbent material;
determining, by the controller, one or more electrical property values based on the one or more voltages;
determining, by the controller, a presence of moisture in the adsorbent material based on the one or more electrical property values;
determining, by the controller, a net change ratio based on the one or more electrical property values and an initial electrical impedance or resistance value that is associated with the adsorbent material in a complete dry state;
determining the net change ratio is greater than at least one threshold of one or more thresholds; and
generating, by the controller, a status message based a status of the adsorbent material that is associated with the at least one threshold.
